# EUROPEAN PATENT APPLICATION

(11) **EP 0 360 072 A1**
(43) Date of publication of application: **28.03.1990**
(21) Application number: 89116491.5
(22) Date of filing: 07.09.1989
(51) Int. Cl.: C07D 413/04, C07D 207/452, A01N 43/34

(54) **Herbicidally active dimethylmaleinimides**

(30) Priority: 20.09.1988 JP 233549/88
(71) Applicant: NIHON TOKUSHU NOYAKU SEIZO K.K., Chuo-ku Tokyo 103 (JP)
(72) Inventor: Kume, Toyohiko, Hino-shi Tokyo (JP); Goto, Toshio, Machida-shi Tokyo (JP); Kamochi, Atsumi, Hino-shi Tokyo (JP); Yanagi, Akihiko, Oume-shi Tokyo (JP); Miyauchi, Hiroshi, Hachioji-shi Tokyo (JP); Shibuya, Katsuhiko, Hachioji-shi Tokyo (JP); Asami, Tadao, Tama-shi Tokyo (JP)
(74) Representative: Schumacher, Günter, Dr.

(57) **Abstract**

Novel dimethylmaleinimides of the formula (I) 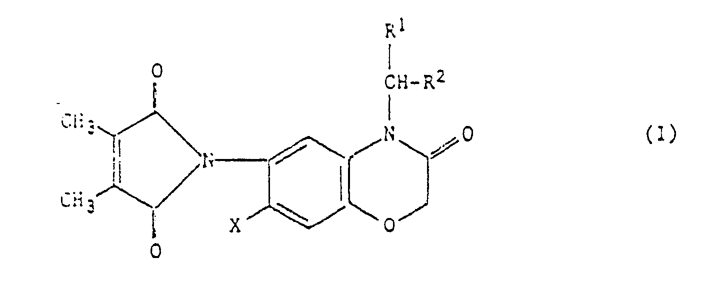 and the use of the new compounds as herbicides.

## Description

The present invention relates to novel dimethylmaleinimides, to processes for their preparation, and to their use as herbicides.

It has already been disclosed that a certain group of tetrahydrophthalimides exhibits herbicidal activities (see Japanese Patent Laid-open No. 30,586/1986 corresponding to EP-A 170,191, US-P 4,640,707 and US-P 4,792,605 respectively) and also that a certain group of dialkylmaleinimides exhibits herbicidal activities (see Japanese Patent Laid-open No. 14,782/1988 corresponding to EP-A 255,601 and US-P 4,792,784).

There have now been found novel dimethylmaleinimides of the formula (I) 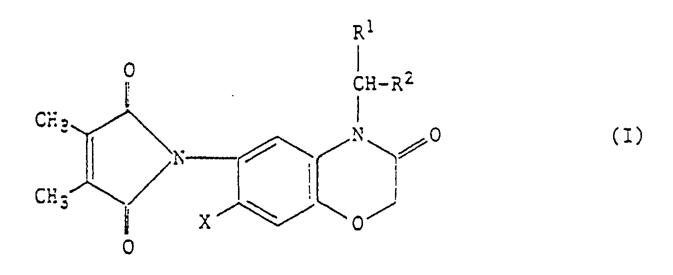 wherein X is hydrogen atom or fluorine atom.
R¹ is hydrogen atom or an alkyl group having 1 to 3 carbon atoms,
R² is a haloalkyl group having 1 to 2 carbon atoms, a halovinyl group, an alkoxyalkoxyalkyl group having 3 to 6 carbon atoms, carbamoyl group, thiocarbamoyl group,

- 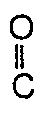 -R³, 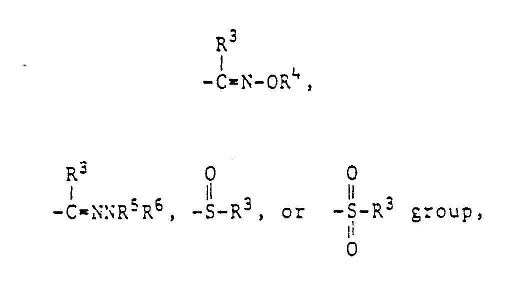 wherein
R³ is an alkyl group having 1 to 4 carbon atoms or a cycloalkyl group having 3 to 6 carbon atoms,
R⁴ is hydrogen atom, an alkyl group having 1 to 4 carbon atoms, an alkenyl group having 2 to 4 carbon atoms, an alkynyl group having 2 to 4 carbon atoms or an alkoxyalkyl group having 2 to 4 carbon atoms, and
R⁵ and R⁶ each are an alkyl group having 1 to 4 carbon atoms,

Dimethylmaleinimides of the formula (I) can be obtained when
a) compounds of the formula (II) 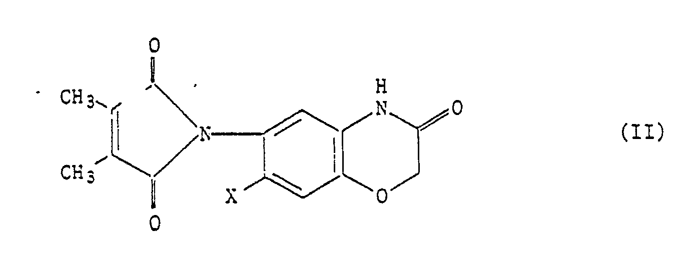 wherein X has the same meaning as above,
   are reacted with compounds of the formula (III) 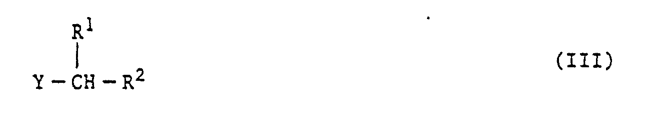 wherein R¹ and R² have the same meanings as above, and
   Y represents halogen atom or -OSO₂-T, wherein T is methyl, phenyl or tolyl group,
   in the presence of inert solvents and if appropriate, in the presence of a base,
   or
b) compounds of the formula (IV) 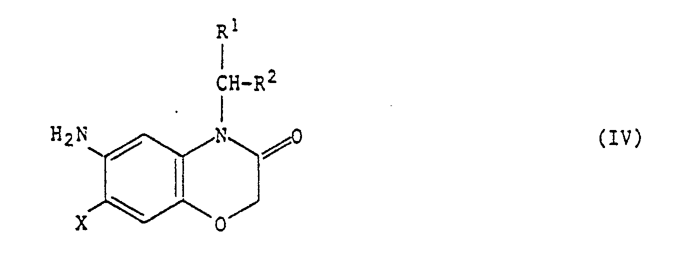 wherein R¹, R² and X have the same meanings as mentioned above, are reacted with 2,3-dimethylmaleic anhydride in the presence of inert solvents,
   or
c) in the case where R² is 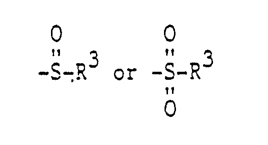 compounds of the formula (V) 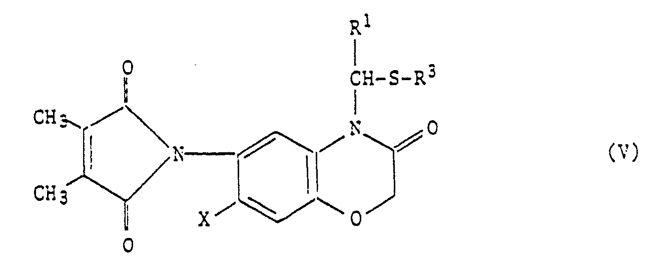 wherein R¹, R³ and X have the same meanings as mentiond above, are oxidized,
   or
d) in the case where R² is thiocarbamoyl compounds of the formula (VI) 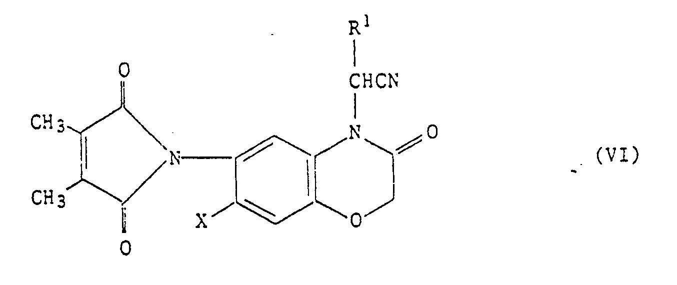 wherein R¹ and X have the same meanings as mentioned above, are reacted with hydrogen sulfide in the presence of inert solvents,
   or
e) in the case where R² is 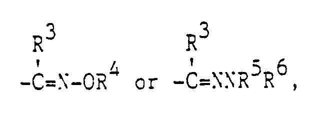 compounds of the formula (VII) 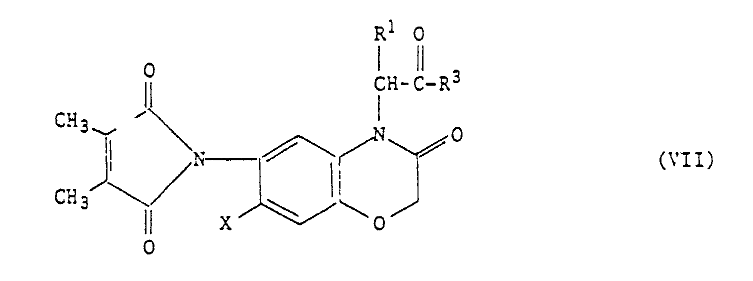 wherein R¹, R³ and X have the same meanings as mentioned above, are reacted with compounds of the formula (VIII)
   H₂N-R⁷ (VIII)
   wherein R⁷ is -OR⁴ or -NR⁵R⁶ and R⁴, R⁵ and R⁶ have the same meaning as mentioned above,
   in the presence of inert solvents.

The dimethylmaleinimides, according to the invention of the formula (I) exhibit powerful harbicidal properties.

Surprisingly, the dimethylmaleinimides exhibit a substantially greater herbicidal action than those known from the prior arts hereinbefore, in particular against upland weeds and also good compatibility to crops.

Among the dimethylmaleinimides according to the invention, of the formula (I), preferred compounds are those in which
X is hydrogen atom or fluorine atom,
R¹ is hydrogen atom or methyl group,
R² is 2-chloroethyl, chlorovinyl, methoxyethoxymethyl, carbamoyl, thiocarbamoyl, 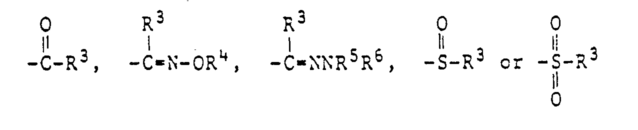 group, wherein R³ is methyl, ethyl or cyclopropyl group,
R⁴ is hydrogen atom, methyl, ethyl, allyl, propargyl or methoxymethyl group, and
R⁵ and R⁶ each are methyl, ethyl, n- or iso-propyl.

Very particularly preferred dimethylmaleinimides of the formula (I) are those in which
X is fluorine atom,
R¹ is hydrogen atom or methyl group,
R² is 1-chlorovinyl, methoxyethoxymethyl, thiocarbamoyl, 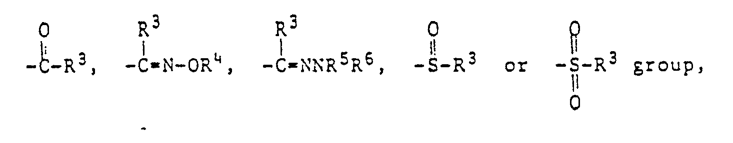 wherein R³ is methyl, ethyl or cyclopropyl group,
R⁴ is hydrogen atom, methyl, ethyl, allyl, propargyl or methoxymethyl group, and
R⁵ and R⁶ each are methyl, ethyl or isopropyl group.

Specifically, the following compounds, according to the invention may be enumerated:

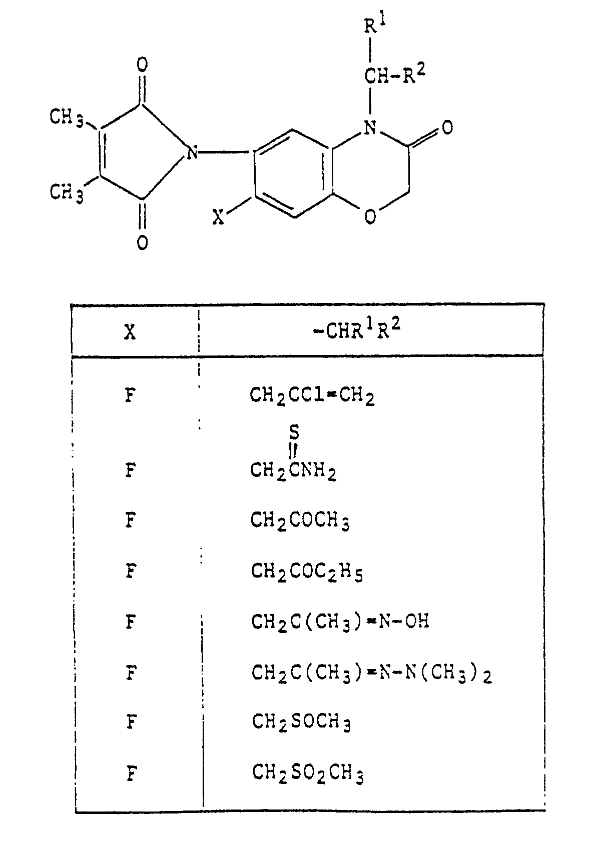

When in the above-mentioned process (a), use is made, as starting materials, of 6-(3,4-dimethylmaleinimido)-7-fluoro-2H-1,4-benzoxazin-3(4H)-one and chloroacetone, for example, the reaction can be expressed as follows:

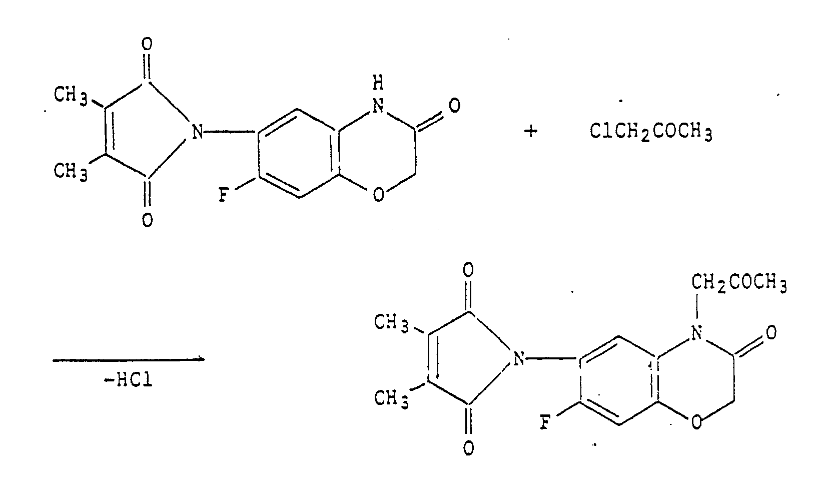

When in the above-mentioned process (b), use is made, as starting materials, of 6-amino-4-(2-chloroally)-7-fluoro-2H-1,4-benzoxazin-3(4H)-one and 2,3-dimethylmaleic anhydride, for example, the reaction can be expressed as follows:

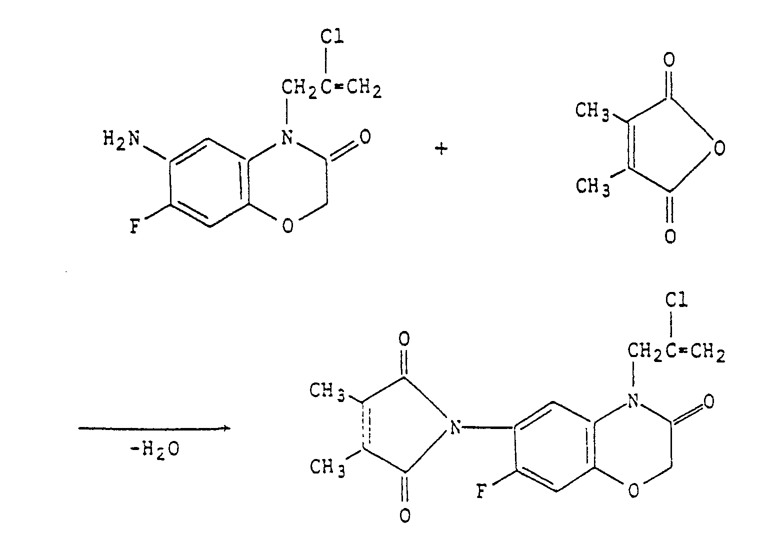

When in the above-mentioned process (c), use is made, as starting materials, of 6-(3,4-dimethylmaleinimido)-7-fluoro-4-methylthiomethyl-2H-1,4-benzoxazin -3(4H)-one and hydrogen peroxide, for example, the reaction can be expressed as follows:

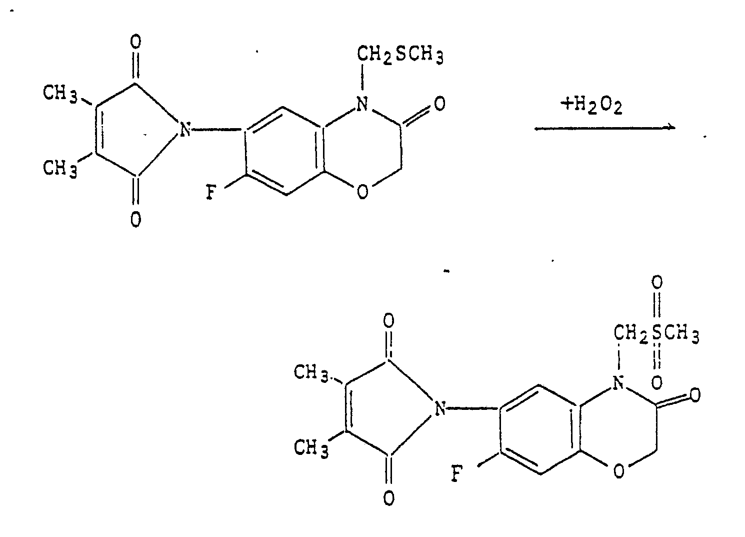

When in the above-mentioned process (d), use is made, as starting materials, of 4-cyanomethyl-6-(3,4-dimethylmaleinimido)-7-fluoro-2H-1,4-benzoxazin-3(4H)-one and hydrogen sulfide, for example, the reaction can be expressed as follows:

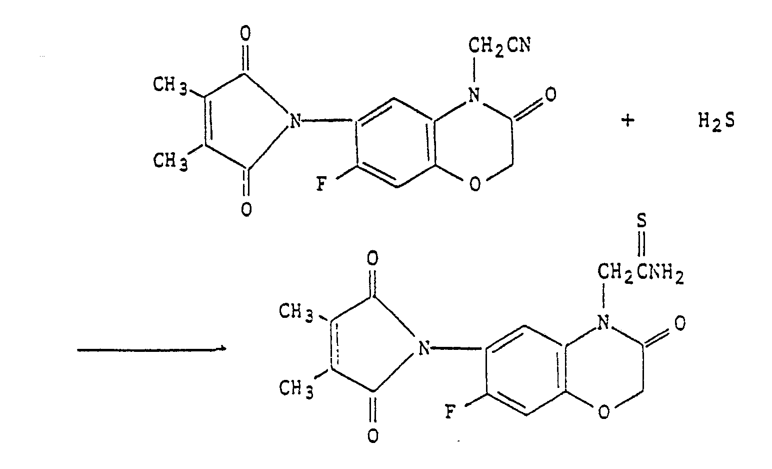

When in the above-mentioned process (e), use is made, as starting material, of 6-(3,4-dimethylmaleinimido )-7-fluoro-4-(2-oxopropyl)-2H-1,4-benzoxazin -3-(4H)-one and methoxy amine, for example, the reaction can be expressed as follows:

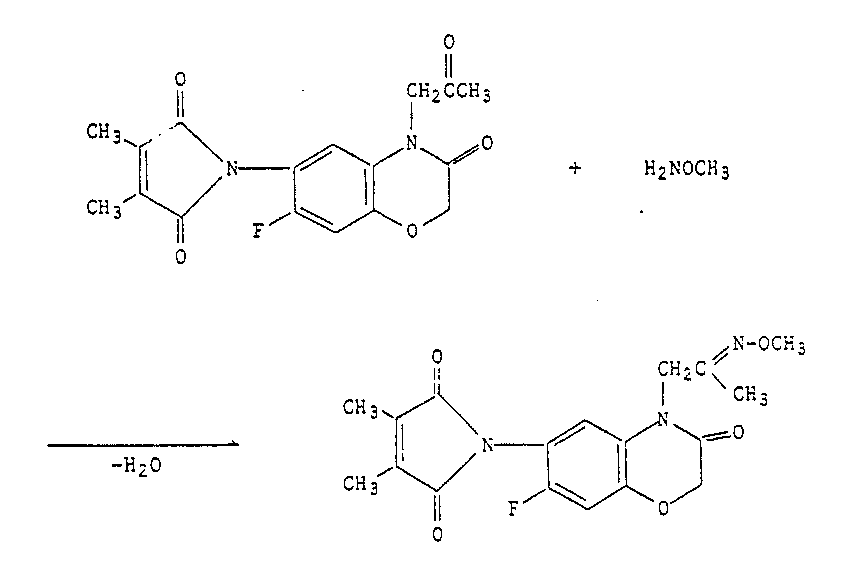

In the above-mentioned process (a), the compounds of the formula (II) are known in themselves as disclosed by U.S. Patent No. 4729784.

The compound (II) can be obtained by process (f) wherein compounds of the formula (IX) 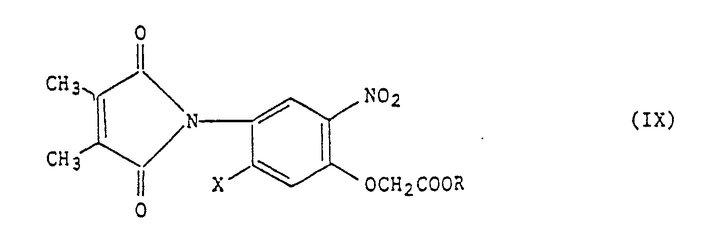 wherein X has the same meaning as above, and R is hydrogen atom or an alkyl group, preferably with 1 to 4 carbon atoms, are reacted with stannous chloride II hydrate in concentrated hydrochloric acid.

The compounds of the formula (IX) are novel in themselves and can be obtained by process (g) wherein compounds of the formula (X) 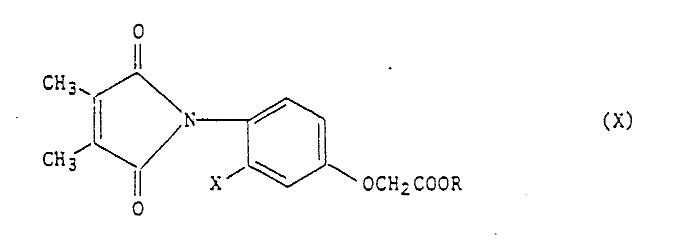 wherein X and R have the same meanings as mentioned above, are nitrated.

The compounds of the formula (X) can be obtained by process (h) wherein compounds of the formula (XI) 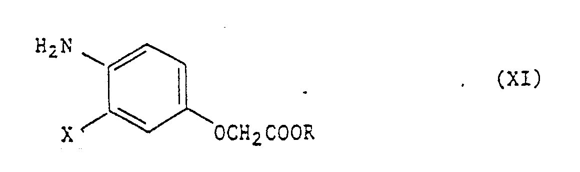 wherein X and R have the same meanings as mentioned above, ar reacted with 2,3-dimethylmaleic anhydride.

The compounds of the formula (XI) can be obtained by process (i), wherein compounds of the formula (XII) 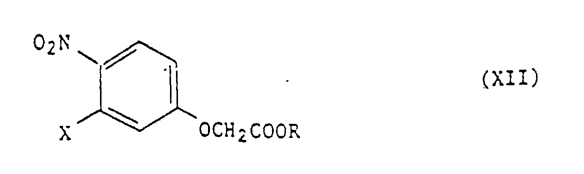 wherein X and R have the same meanings as mentiond above, are reduced.

The compounds of the formula (XII) can be obtained by process (j), wherein compounds of the formula (XIII) 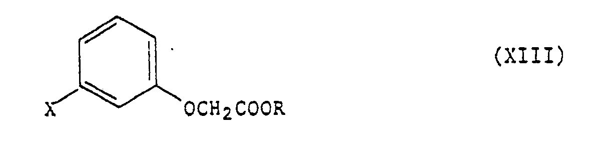 wherein X has the same meaning as above, are nitrated.

The compounds of the formula (XIII) can be obtained by process (k), wherein compounds of the formula (XIV) 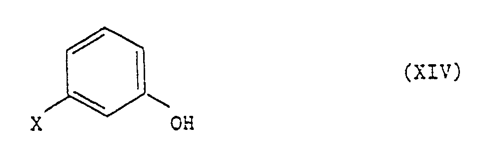 wherein X has the same meaning as mentioned above, are reacted with compounds of the formula (XV)
BrCH₂COOR (XV)
wherein R has the same meaning as above.

The compounds of the formula (XIV) are known in themselves and, as specific examples, may be mentioned phenol and 3-fluorophenol.

The compounds of the formula (XV) are known in themselves and, as specific examples, may be mentioned bromoacetic acid and ethyl bromoacetate.

In carrying out the above-mentioned process (f), as will be stated in the following examples, about 3 mols of stannous chloride II hydrate is reacted with one mol of the compounds of the formula (IX) in a concentrated hydrochloric acid solution.

In carrying out the above-mentioned process (g), as will be stated in the following example, the compounds of the formula (X) is reacted, according to the conventional nitration reaction, with a mixture of 98% nitric acid and a 95% concentrated sulfuric acid so as to easily obtain the aimed compounds.

In carrying out the above-mentioned process (h), the compounds of the formula (XI) can be obtained by successively carrying out the above-mentioned processes (k), (j), (i) and (h) as will be illustrated in the following referential example.

The reaction partner, viz., the compounds of the formula (III) employed in the above-mentioned process (a) have the substituents as defined under R¹, R² and Y referring to the compounds of the formula (III), especially those defined as preferable substituents and, among others, Y preferably denotes chlorine atom, bromine atom or tosyloxy group.

The compounds of the formula (III) include known alkylation agent and can be exemplified in the following:
1,3-dichloropropene,
2,3-dichloropropene,
chloroacetone,
1-chloro-2-butanone,
3-chloro-2-butanone,
2-chloro-3-pentanone,
chloromethyl cyclopropyl ketone.

Referring to the compounds of the formula (IV) employed in the process (b) mentioned above, the respective symbols R¹, R² and X have the same meanings as mentioned above and, especially, have those defined therein as preferable ones.

The compounds of the formula (IV) can be easily obtained according to the teachings given in Japanese Patent Laid-open No. 14782/1988.

As specific examples, the following compounds are included:
4-(2-oxopropyl)-6-amino-2H-1,4-benzoxazin-3(4H)-one,
4-(2-oxopropyl)-6-amino-7-fluoro-2H-1,4-benzoxazin-3(4H)-one, and
4-(2-chloro-2-propenyl)-6-amino-7-fluoro-2H-1,4-benzoxazin-3(4H)-one.

The compounds of the formula (V) employed in the above-mentioned process (c) are included in the formula (I) of the present invention and can be obtained by the above-mentioned process (a) or (b).

The compounds of the formula (VI) employed in the above-mentioned process (d) are known (see Japanese Patent Laid-open No. 14782/1988), and as specific examples, the following compounds are included:
3-(cyanomethyl)-6-(3,4-dimethylmaleinimido)-2H-1,4-benzoxazin-3(4H)-one
3-(cyanomethyl)-6-(3,4-dimethylmaleinimido)-7-fluoro-2H-1,4-benzoxazin-3(4H)-one,
3-(1-cyanoethyl)-6-(3,4-dimethylmaleinimido)-7-fluoro-2H-1,4-benzoxazin-3(4H)-one.

The compounds of the formula (VII) employed in the above-mentioned process (e) are included in the formula (I) of the present invention and can be obtained by the above-mentioned process (a) or (b).

Similarly, the compounds of the formula (VIII) employed in the above-mentioned process (e) are well known in the field of organic chemistry.

In carrying out the above-mentioned process (a), any kind of inert organic solvents can be used.

Examples of the diluents are nitriles such as acetonitrile; dimethylformamide; dimethylsulfoxide; sulfolane, ketones such as acetone, methyl ethyl ketone, methyl isobutyl ketone; ethers such as tetrahydrofuran, dioxane.

The process (a) may be conducted in the presence of a dehydrohalogenation agent.

Examples of such dehydrohalogenation agent are sodium carbonate, potassium carbonate, and sodium hydride.

The reaction temperature of the process (a) can be varied within a substantial range. In general, the reaction can be carried out at a temperature of about 10 to 150°C, preferably a temperature of about 30 to 110°C.

It is preferred to carry out the reaction under the normal pressure, although higher or reduced pressure can also be used.

In carrying out the process (a), about 1 to 1.2 moles of the compounds of the formula (III) as alkylating agent may be employed per mole of the compounds of the formula (II). These compounds are reacted with each other in an inert solvent and in the presence of a dehydrohalogenation agent, so that the aimed compounds of the formula (I) can be obtained.

As appropriate diluents in carrying out the process (b), any of inert solvents can be used.

Examples of such solvents include aromatic hydrocarbons such as, for example, toluene, xylene, chlorobenzene, dichlorobenzene, biphenyl; ethers such as, for example, dioxane, diphenyl ether; organic acids such as, for example, acetic acid, propionic acid, etc.

The process (b) may be carried out substantially within a wide range of temperatures. It may be carried out at a temperature, for example, between about 70°C and about 280°C, preferably between about 80°C and about 100°C.

The reaction can preferably be carried out under the normal pressure, but it may be operated under a higher or a reduced pressure.

In carrying out the process (b), for example, about 1 to 1.2 moles of 2,3-dimethylmaleic anhydride per mole of the compounds of the formula (IV) are employed to react them with each other in the presence of an inert solvent to obtain the aimed compounds of the formula (I).

Apart from the reaction conditions employed in the above-mentioned process (b), the compounds of the formula (I) can be obtained by subjecting the compounds of the formula (IV) and 2,3-dimethylmaleic anhydride to dehydrocyclization reaction with the use of a catalyst such as toluene sulfonic acid, benzene sulfonic acid or sulfuric acid in the presence of an inert solvent boiling at or higher than 150°C such as dichlorobenzene, diphenyl ether, Dowtherm (an eutectic mixture of biphenyl and diphenyl ether) etc.

In carrying out the process (c), use may be made of oxidation reaction with the use of an organic peroxide such as, for example, m-chloroperbenzoic acid in the presence of chloroform or carbon tetrachloride but a more simple reaction is to use hydrogen peroxide solution as oxidizing agent in the presence of an organic acid such as, for example, acetic acid.

In case of oxidation to the sulfoxide compounds, one mole of the compounds of the formula (V) is treated with 1 to 1.4 moles of hydrogen peroxide solution at a temperature not higher than 40°C and in the presence of acetic acid, so as to easily obtain the aimed sulfoxide compounds of the formula (I) wherein n=0.

In case of oxidation to the sulfone compounds, one mole of the compounds of the formula (V) is treated with 2 to 2.8 moles of hydrogen peroxide solution under reflux in the presence of acetic acid so as to easily obtain the aimed sulfone compounds of the formula (I).

In carrying out the process (d), use is made, as appropriate diluents, of any of inert solvents.

Examples of such solvents may include hydrocarbons such as benzene, toluene and like, ethers such as ethyl ether, tetrahydrofuran, dioxane and like, and pyridine.

The reaction temperature of the process (d) can be varied within a substantial range. In general, the reaction can be carried out at a temperature of about 0 to 100°C, preferably a temperature of about 20 to 80°C.

It is preferred to carry out the reaction under the normal pressure, although a higher or reduced pressure can also be used.

In carrying out the process (d), the compounds of the formula (VI) are saturated with hydrogen sulfide in the presence of one of the above-mentioned inert solvents as well as an organic base such as triethyl amine and like so as to obtain the aimed compounds of the formula (I).

In carrying out the process (e), use may be made, as appropriate diluent, of any of inert solvents. Examples of such solvents include water, alcohols such as methanol, ethanol, etc., ethers such as tetrahydrofurane, dioxanes.

The reaction temperature of the process (e) can be varied within a substantial range. In general, the reaction is carried out at a temperature of about 10 to 100°C, preferably a temperature of about 30 to 80°C.

It is preferred to carry out the reaction under the normal pressure, although a higher or reduced pressure can also be used.

In carrying out the process (e), about 1 to 1.2 moles of the compounds of the formula (VIII) may be employed per mole of the compound of the formula (VII). These compounds are reacted with each other in an inert solvent, so that the aimed compounds of the formula (I) can be obtained.

The active compounds according to the invention can be used as defoliants, desiccants, agents for destroying broad-leaved palnts and, especially, as weedkillers. By weeds, in the broadest sense, there are to be understood all plants which grow in locations where they are undesired. Whether the substances according to the invention act as total or selective herbicides depends essentially on the amount used.

The active compouns according to the invention can be used, for example, in connection with the following plants:
Dicotyledon weeds of the genera: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver and Centaurea.
Dicotyledon cultures of the genera: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis and Cucurbita.
Monocotyledon weeds of the genera: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus and Apera. Monocotyledon cultures of the genera: Oryza, Zea, Triticum, Hordeum, Avena, Secale, Sorghum, Panicum, Saccharum, Ananas, Asparagus and Allium.

However, the use of the active compounds according to the invention is in no way restricted to these genera, but also extends in the same manner to other plants.

The compounds are suitable, depending on the concentration, for the total combating of weeds, for example on industrial terrain and rail tracks, and on paths and squares with or without tree plantings. Equally, the compounds can be emplyed for combating weeds in perennial cultures, for example afforestations, decorative tree plantings, orchards, vineyards, citrus groves, nut orchards, banana plantations, coffee plantations, tea plantations, rubber plantations, oil palm plantations, cocoa plantations, soft fruit plantings and hopfields, and for the slective combating of weeds in annual cultures.

The active compounds can be converted into the customary formulations, such as solutions, emulsions, wettable powders, suspensions, powders, foams, pastes, granules, aerosols, natural and synthetic materials impregnated with active compound, very fine capsules in polymeric substances, coating compositions for use on seed, and formulations used with burning equipment, such as fumigating cartridges, fumigating cans and fumigating coils, as well as ULV cold mist and warm mist formulations.

These formulations may be produced in known manner, for example by mixing the active compounds with extenders, that is to say liquid or liquefied gaseous or solid diluents or carriers, optionally with the use of surface-active agents, that is to say emulsifying agents and/or dispersing agents and/or foam-forming agents. In the case of the use of water as an extender, organic solvents can, for example, also be used as auxiliary solvents.

As liquid solvents diluents or carriers, there are suitable in the main, aromatic hydrocarbons, such as xylene, toluene or alkyl napthalenes, chlorinated aromatic or chlorinated aliphatic hydrocarbons, such as chlorobenzenes, chloroethylenes or methylene chloride, aliphatic hydrocarbons, such as cyclohexane or paraffins, for example mineral oil fractions, alcohols, such as butanol or glycol as well as their ethers and esters, ketones, such as acetone, methyl ethyl ketone, methyl isobutyl ketone or cyclohexanone, or strongly polar solvents, such as dimethylformamide and dimethyl-sulphoxide, as well as water.

By liquefied gaseous diluents or carriers are meant liquids which would be gaseous at normal temperature and under normal pressure, for example aerosol propellants, such as halogenated hydrocarbons as well as butane, propane, nitrogen and carbon dioxide.

As solid carriers there may be used ground natural minerals, such as kaolins, clays, talc, chalk, quartz, attapulgite, montmorillonite or diatomaceous earth, and ground synthetic minerals, such as highly-dispersed silicic acid, alumina and silicates. As solid carriers for granules there may be used crushed and fractionated natural rocks such as calcite, marble, pumice, sepiolite and dolomite, as well as synthetic granules of inorganic and organic meals, and granules of organic material such as sawdust, coconut shells, maize cobs and tobacco stalks.

As emulsifying and/or foam-forming agents there may be used non-ionic and anionic emulsifiers, such as polyoxyethylene-fatty acid esters, polyoxyethylene-fatty alcohol ethers, for example alkylaryl polyglycol ethers, alkyl sulphonates, alkyl sulphates, aryl sulphonates as well as albumin hydrolysis products. Dispersing agents include, for example, lignin sulphite waste liquors and methylcellulose.

Adhesives such as carboxymethylcellulose and natural and synthetic polymers in the form of powders, granules or latices, such as gum arabic, polyvinyl alcohol and polyvinyl acetate, can be used in the formulation.

It is possible to use colorants such as inorganic pigments, for example iron oxide, titanium oxide and Prussian Blue, and organic dyestuffs, such as alizarin dyestuffs, azo dyestuffs or metal phthalocyanine dyestuffs, and trace nutrients, such as salts of iron, manganese boron, copper, cobalt, molybdenum and zinc.

The formulations in general contain from 0.1 to 95 per cent by weight of active compound, preferably from 0.5 to 90 per cent by weight.

The active compounds according to the invention, as such or in the form of their formulations, can also be used, for combating weeds, as mixtures with known herbicides, finished formulations or tank mixes being possible.

Mixtures with other known active compounds, such as herbicides, fungicides, insecticides, acaricides, nematicides, bird repellants, plant nutrients and agents which improve soil structure, are also possible.

The active compounds can be used as such, in the form of their formulations or in the use forms prepared therefrom by further dilution, such as ready-to-use solutions, suspensions, emulsions, powders, pastes and granules. They are used in the customary manner, for example by watering, spraying, atomising or scattering.

The active compounds according to the invention can be applied either before or after emergence of the plants.

They can also be incorporated into the soil before sowing. They are used, in particular, after emergence of the plants.

The amount of active compound used can vary within a substantial range. It depends essentially on the nature of the desired effect. In general, the amounts used are between 0.0001 and 3 kg of active compound per hectare of soil surface, preferably between 0.001 and 2 kg per ha.

The preparation and use of the active compounds according to the invention can be seen from the following examples.

### Preparative Examples:

### Example 1

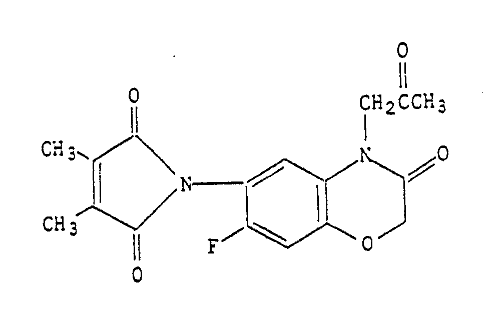

A mixture composed of 6-(3,4-dimethylmaleinimido)-7-fluoro-2H-1,4-benzoxazin-3(4H)-one (2.9 g) and acetonitril (100 ml) was stirred under heating at a temperature in the range of 60 to 70°C and, after the complete dissolution having been confirmed, potassium carbonate (3 g) was added to the solution.

To the mixture was gradually added dropwise chloroacetone (1.1 g) over an hour. After the completion of the dropwise addition, the reaction mixture was stirred at a temperature in the range of 60° to 70°C for two hours and then cooled to room temperature. The cooled reaction mixture was filtered. The filtrate and the washings were combined and concentrated under reduced pressure to obtain a solid material. The remaining unreacted starting material was removed by using a basic alumina column (n-hexane/ethyl acetate = 3/1) thereby leaving crude crystalls, which were recrystallized from a mixture of n-hexane-ethyl acetate to obtain the aimed 6-(3,4-dimethylmaleinimido)-7-fluoro-4-(2-oxopropyl)-2H-1,4-benzoxazin-3(4H)-one (2.4 g) having a melting point in the range of 200 to 210°C.

### Example 2

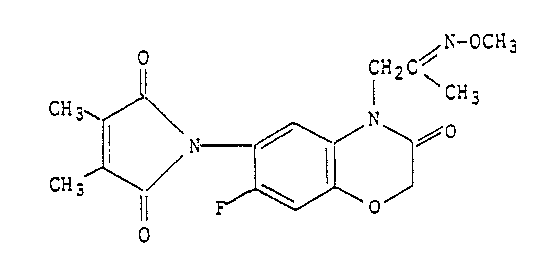

A mixture composed of 6-(3,4-dimethylmaleinimido)-7-fluoro-4-(2-oxopropyl)-2H-1,4-benzoxazin-3(4H)-one (0.82 g), methoxyamine hydrochloride (about 0.4 g) and methanol (50 ml) was heated under reflux for an hour. The reaction mixture was cooled to room temperature and thereafter was concentrated under reduced pressure, while the residue was refined by a silica-gel column (n-hexane/ethyl acetate = 3/1) to obtain crude crystals which were recrystallized from a mixture of n-hexane-ethyl acetate, thereby to obtain the aimed 6-(3,4-dimethyl maleinimido)-7-fluoro-4(2-methoxyiminopropyl)-2H-1,4-benzoxazin-3(4H)-one (0.52 g) in white crystals. The melting point of the product was in the range of 142 to 144°C.

### Example 3

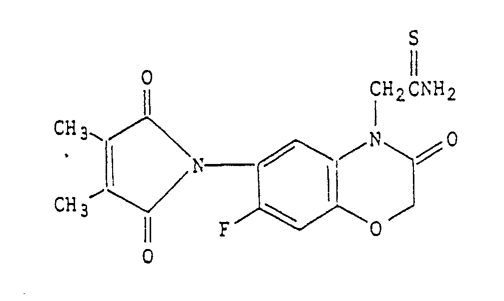

To a mixture consisting of 4-cyanomethyl-6-(3,4-dimethyl maleinimido)-7-fluoro-2H-1,4-benzoxazin -3(4H)-one (2 g), triethylamine (0.62 g) and tetrahydrofuran (50 ml) was added hydrogen sulfide at a temperature from 20° to 30°C to saturation. After stirring at a temperature of 20° to 30°C for eight hours, low-boiling substance was distilled off under reduced pressure and the thus obtained residue was refined by a silica-gel column (chloroform/ethanol = 5/1), thereby to obtain the aimed 6-(3,4-dimethylmaleinimido)-7-fluoro- 4- thiocarbamoylmethyl-2H-1,4-benzoxazin -3(4H)-one (1.0 g) having a melting point as a porous solid.

### Example 4

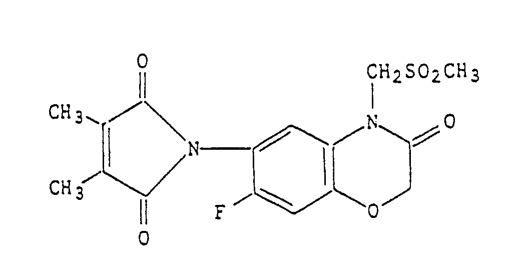

To a mixture consisting of 6-(3,4-dimethylmaleinimido)-7-fluoro-4-methylthiomethyl-2H-1,4-benzoxazin -3(4H)-one (0.7 g) and acetic acid (20 g) was added dropwise a 35% hydrogen peroxide solution (0.43 g) at a temperature in the range of 20° to 30°C. After the dropwise addition mentioned above, acetic acid was further added to the mixture to form a solution-like reaction mixture,that was heated under reflux for five hours. The residue obtained by evaporating the reaction mixture under reduced pressure was dissolved in ethyl acetate, followed by washing with saturated aqueous solution of sodium bicarbonate, water and saturated aqueous solution of sodium chloride, in that order and drying over anhydrous sodium sulfate. By removing ethyl acetate by distillation was obtained the aimed 6-(3,4-dimethylmaleinimido)-7-fluoro-4-methylsulfonylmethyl-2H-1,4-benzoxazin-3(4H)-one (0.75 g) in a porous solid.

### Example 5

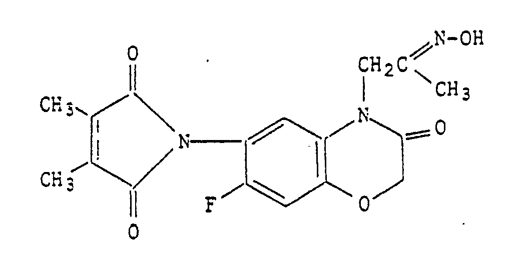

A mixture composed of 6-(3,4-dimethylmaleinimido)-7-fluoro-4-(2-oxopropyl)-2H-1,4-benzoxazin-3(4H)-one (0.5 g),hydroxylamine hydrochloride (0.3 g) and methanol (50 ml) was heated under reflux for one hour. After cooling the solution to room temperature, it was concentrated under reduced pressure, and the residue was dissolved in dilute hydrochloric acid followed by three times extractions thereof with ethyl acetate (50 ml). The organic layer obtained was washed with aqueous sodium bicarbonate solution and dried over anhydrous sodium sulfate, followed by concentration under reduced pressure to obtain crude crystals which were further recrystallized with a mixture of chloroform/methanol/hexane to obtain the aimed 6-(3,4-dimethylmaleinimido)-7-fluoro-4-(2-hydroxyiminopropyl)-2H-1,4-benzoxazin-3(4H)-one (0.32 g) having a melting point in the range of 225 to 226°C.

Together with the above-prepared compounds, other compounds according to the present invention which can be obtained according to each of the above-mentioned exemplified procedures are given in the following Table I:

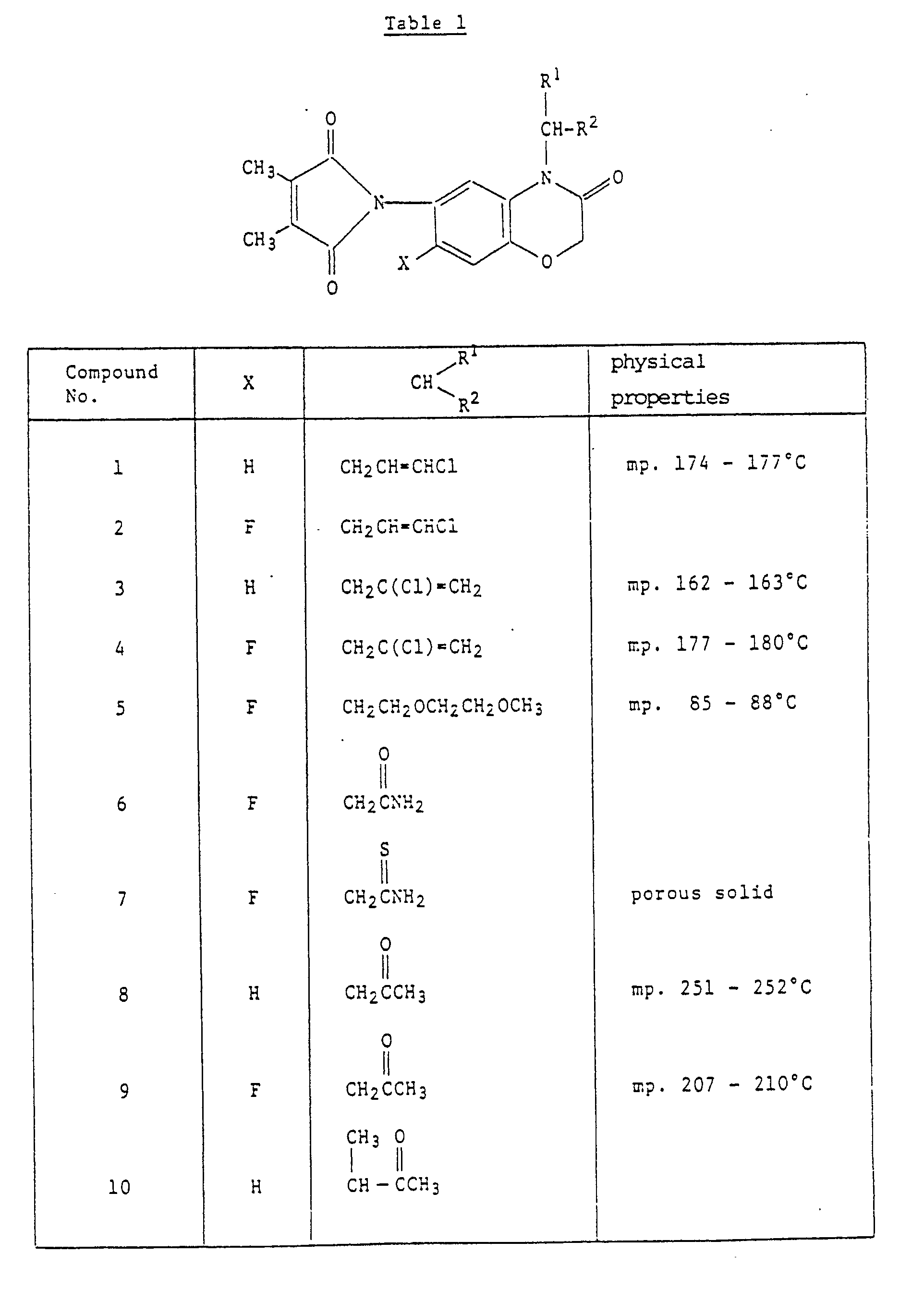

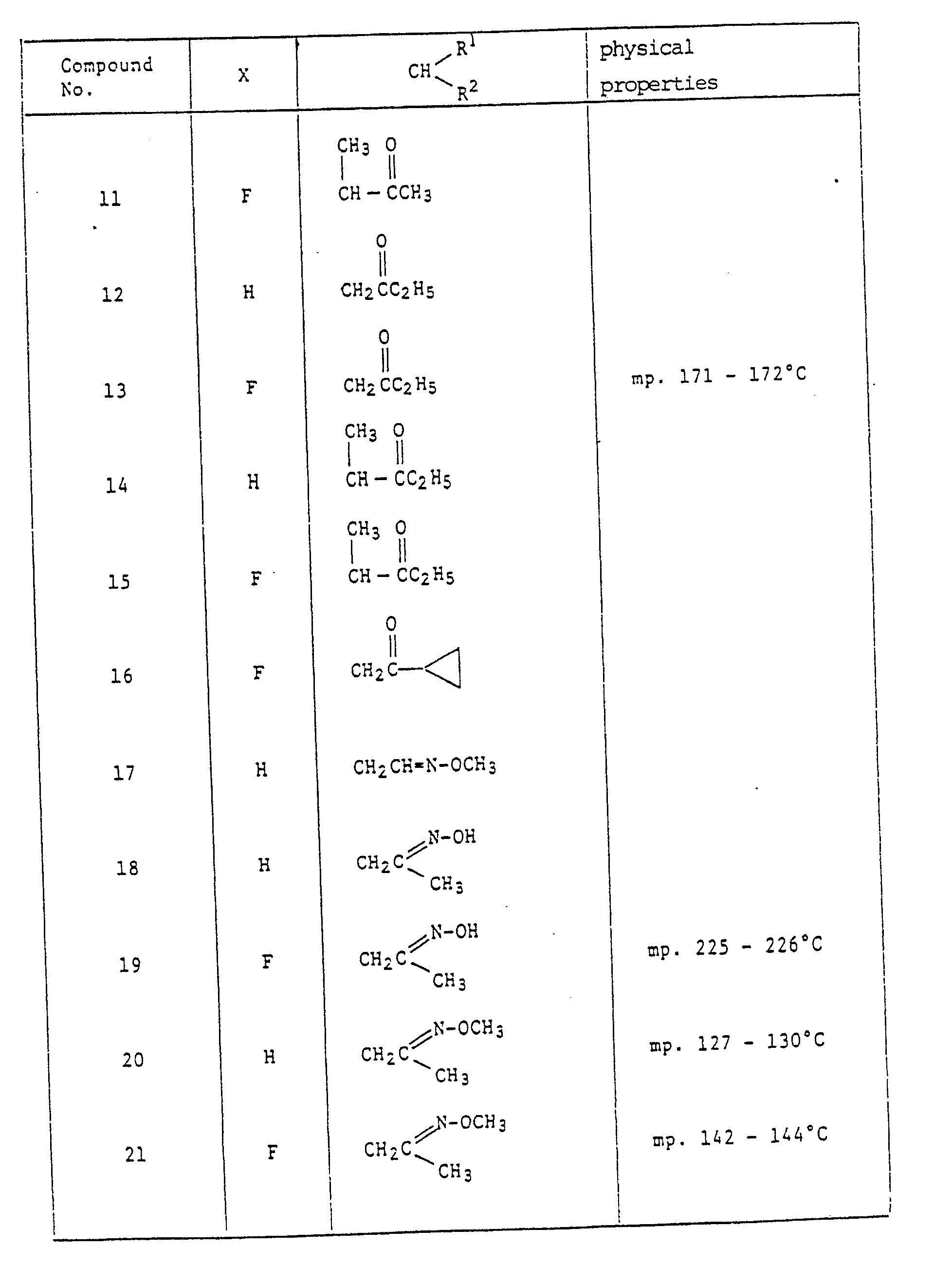

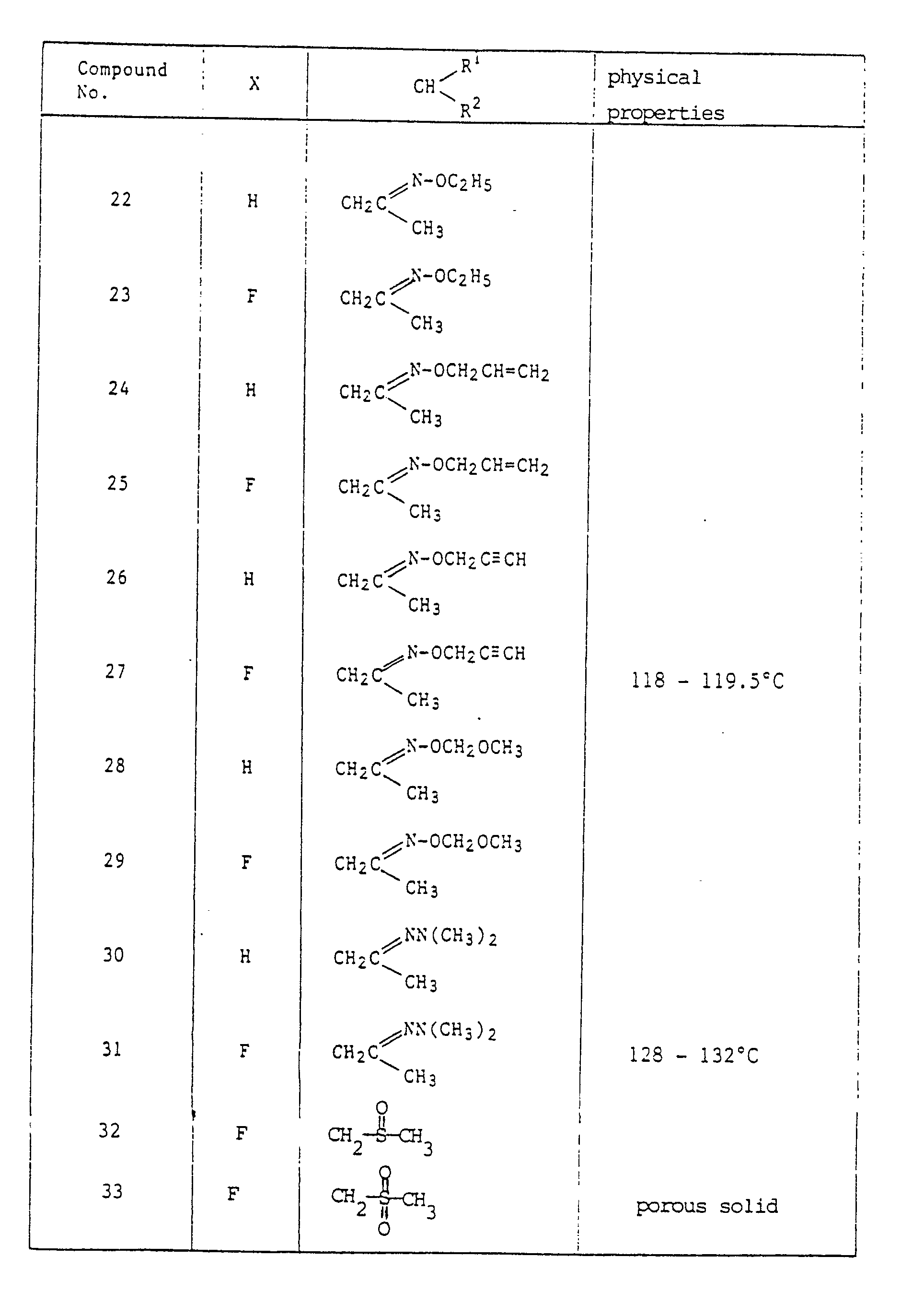

### Example 6 (production of intermediate compound)

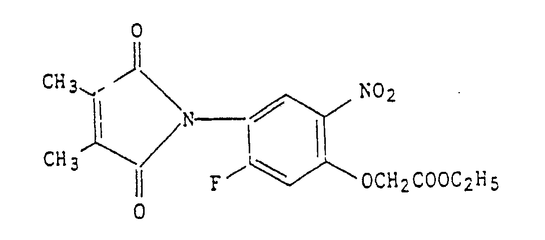

N-(4-ethoxycarbonylmethoxy-2-fluorophenyl)-3,4-dimethyl maleinimide (4.4 g) was added to a 95% concentrated sulfuric acid (100 g) at 0 - 5°C and, after a thorough agitation thereof, a 98% nitric acid (1 g) was dropwise added thereto.

After a two-hours agitation of the reaction mixture at 0 to 5°C, it was poured into water to separete crystals that were in turn extracted with toluene (450 ml). The extract was washed with water, a saturated aqueous solution of sodium bicarbonate and water, in that order, and dried over anhydrous sodium sulfate. The solid residue obtained by evaporating the toluene from the reaction mixture, was recrystallized from a solvent (toluene/hexane) to obtain the aimed N-(4-ethoxy-carbonylmethoxy-2-fluoro-5-nitrophenyl)-3,4-dimethylmaleinimide (4.46 g) having a melting point in the range of 145 to 146°C.
In the same way as the above Example 6, N-(4-methoxycarbonyl-methoxy-2-fluoro-5-nitrophenyl)-3,4-dimethylmaleimide and N-(4-iso-propoxycarbonyl-methoxy-2-fluoro-5-nitrophenyl)-3,4-dimethylmaleimide can be obtained.

### Example 7 (production of intermediate compound)

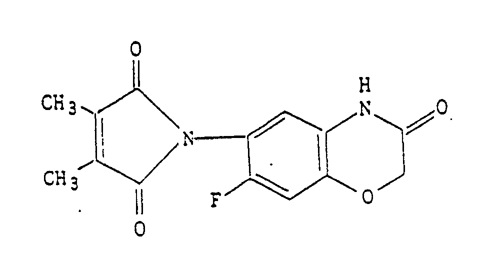

To a suspension in ethanol (200 ml) of N-(4-ethoxycarbonylmethoxy-2-fluoro-5-nitrophenyl)-3,4-dimethylmaleinimide (3.66 g) was added dropwise at 20° to 30°C a solution of stannous chloride II hydrate (0.0286 mols equivalent) in concentrated hydrochloric acid (100 ml). After the dropwise addition mentioned above, the suspension was further agitated at 50° to 60°C continuously for two hours. The major part of ethanol was distilled off from the suspension under reduced pressure to obtain a pasty residue,to which was added water (100 ml) followed by cooling to 10° to 15°C. The resulting solid substance was collected by filtration, washed with iced water, and dried to obtain the aimed 6-(3,4-dimethylmaleinimido )-7-fluoro-2H-1,4-benzoxazin -3(4H)-one (2.2 g). The product was recrystallized from ethanol to obtain the aimed 6-(3,4-dimethylmaleinimido)-7-fluoro-2H-1,4-benzoxazin-3(4H)-one (2.0 g).

### Referential Example

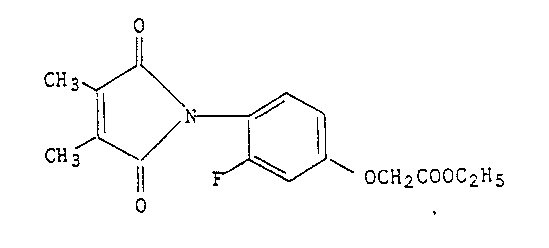

A mixture consisting of 3-fluorophenol (31.2 g), potassium carbonate (37.3 g) and acetonitrile (150 ml) was stirred at 40° - 50°C for 30 minutes, to which was added dropwise at 20 to 30°C ethyl bromoacetate (46.5 g). After a six hours agitation at 70 to 80°C, the reaction mixture was cooled to a temperature in the range of 10 to 20°C and filtered. The filtrate was evaporated under reduced pressure to obtain a residue to which was added ether (200 ml) and water (80 ml) followed by separating ether layer after agitation thereof.

The separated ether layer was washed with cold 5% aqueous potassium hydroxide, water,and saturated aqueous sodium chloride, in that order, and dried over anhydrous sodium sulfate. After removal of the solvent by evaporation, the reaction product was distilled under reduced pressure. Fractions boiling at 150 - 152°C/20 mmHg were collected to obtain ethyl (3-fluorophenoxy) acetate (48.2g), which (48.1 g) was added to 95% sulfuric acid (300 g) at 0 to 5°C, and to the mixture was added dropwise 98% nitric acid (16.7 g) at a temperature in the range of -10 to 0°C. After the dropwise addition mentioned above, the reaction mixture was stirred for an hour at 0 to 5°C, then poured into ice and was extracted with ether.

The extract thus obtained was washed with water, saturated aqueous sodium bicarbonate, water, and saturated aqueous sodium chloride in that order, followed by drying over anhydrous sodium sulfate. The ether was distilled off to obtain an oily residue that was determined by gas chromatography analysis to be a mixture consisting of a trace amount of unreacted ethyl (3-fluorophenoxy) acetate, ethyl (3-fluoro-6-nitrophenoxy)acetate and the aimed intermediate compound, viz., ethyl (3-fluoro-4-nitrophenoxy)acetate. This mixture together with ethanol (150ml) and a 10% palladium carbon (8 g) was charged in an autoclave and subjected to catalytic hydrogenation at an initial pressure of 10 kg/cm² until hydrogen absorption was completed.

To the reaction mixture was added tetrahydrofuran (500 ml) followed by agitation and then filtration. After the filtrate was evaporated under reduced pressure, the residue was dissolved in ethyl acetate (500 ml). The ethyl acetate solution was washed with a 5% aqueous solution of potassium hydroxide, water and saturated solution of sodium chloride in that order and dried over anhydrous sodium sulfate, and then evaporated under reduced pressure to obtain (4-amino-3-fluorophenoxy)acetate (14.8 g). The whole amount of the product was, without any refining procedure, dissolved in acetic acid (100 ml) and treated with 2,3-dimethylmaleic anhdydride (9.2 g) at 20 to 30°C. The reaction mixture was stirred at 20 to 30°C for 30 minutes and then heated under reflux for 2 hours.

After removal of the acetic acid from the reaction mixture by evaporation under reduced pressure, the resulting residue was mixed thoroughly with toluene (300 ml) and filtered. The resulting filter cake was recrystallized from ethanol and determined to be ethyl (4-acetomino-3-fluorophenoxy)-acetate that was an undesirable side product.

The filtrate was washed with a 5% aqueous solution of potassium hydroxide and water in that order, dried over anhydrous sodium sulfate and was freed from the solvent by distillation under reduced pressure, followed by the recrystallization of the resulting soild residue with ethanol so as to obtain the aimed N-(4-ethoxycarbonylmethoxy-2-fluorophenyl)-3,4-dimethylmaleinimide (5.1 g) having a melting point in the range of 105 to 109°C.

### Biotest Examples:

Known comparison compound employed:

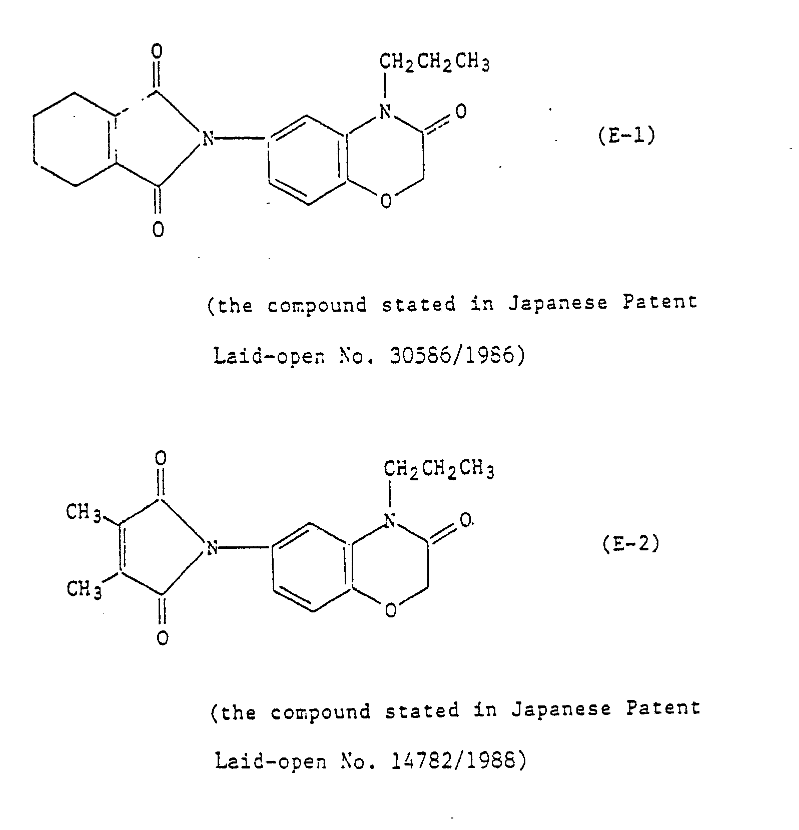

### Example 8

Test on weeds in a flooded paddy by water surface application:-

### Preparation of an active compound formulation

Carrier: 5 parts by weight of acetone
Emulsifier: 1 part by weight of benzyloxy polyglycol ether

A formulation of an active compound was obtained as an emulsifiable concentrate by mixing 1 part by weight of the active compound with the above-mentioned amounts of the carrier and the emulsifying agent. A predetermined amount of the formulation was diluted with water.

### Testing method

Each of several pots, having a size of 25 x 20 x 9 cm and an area of 1/2,000 are, was filled with paddy soil, which was kept under wet conditions.
Rice seedlings (variety: Nihonbare) at the 2.5-leaf stage (plant height:
ca 15 cm) were transplanted into these pots at the rate of 3 plants/stand, with 2 stands/pot.
Then, seeds and tubers of the following weeds were sown in the soil:
barnyard grass (Echinochloa crus-galli);
umbrella plant (Cyperus diformis);
monochoria (Monochoria vaginalis); and
annual broad-leaved weeds such as false pimpernel (Lindernia pyxidaria), toothcup (Rotala indica), American waterwort (Elatine triandra), Red stem (Ammannia multiflora), and dopatrium (Dopatrium junceum Hamilt.).

After 2 days, water was introduced to a depth of 2 - 3 cm above the soil of the pots. Five days after the transplantation of the rice seedlings, the emulsion of the active compound, which had been prepared in the manner mentioned above, was applied to the pots in a predetermined amount by means of a pipette. After that, the water was maintained at a depth of 3 cm through the test period.

Four weeks after the application of the active compound, the degree of damage to the weeds and the degree of phytotoxicity on the paddy-rice plants were determined, and recorded according to the following assessment scale rated from 0 to 5.

| Rating | Herbicidal effect of active compound on weed in % * |
|---|---|
| 5 | 95% or more (fatal effect) |
| 4 | at least 80% and less than 95% |
| 3 | at least 50% and less than 80% |
| 2 | at least 30% and less than 50% |
| 1 | at least 10% and less than 30% |
| 0 | less than 10% (no herbicidal effect) |

| Rating | Phytotoxic effect of active compound on crops in % * |
|---|---|
| 5 | at least 90% (fatal phytotoxicity) |
| 4 | at least 50% and less than 90% |
| 3 | at least 30% and less than 50% |
| 2 | at least 10% and less than 30% |
| 1 | more than 0% and less than 10% |
| 0 | 0% (no phytotoxicity) |

| | |
|---|---|
| * These values (%) are those obtained by comparing the test data in the treated section with the test data in the control (untreated) section. | |

The test results are shown in Table 2.

**Table 2**

| Active compound No. | Amount of active compound (kg/ha) | Herbicidal effect on weeds | | | | Phytotoxic effect on rice plants |
|---|---|---|---|---|---|---|
| | | Barnyard grass | Umbrella plant | Monochoria | Annual broad-leaved grass | |
| 13 | 0.125 | 5 | 5 | 5 | 5 | 1 |
| | 0.06 | 4 | 5 | 4 | 5 | 0 |

| (Known compound) | | | | | | |
|---|---|---|---|---|---|---|
| E-1 | 0.125 | 1 | 4 | 2 | 2 | 2 |
| | 0.06 | 0 | 2 | 1 | 1 | 1 |
| E-2 | 0.125 | 4 | 5 | 4 | 4 | 0 |
| | 0.06 | 2 | 3 | 2 | 3 | 0 |

### Example 9

### Pre-emergence soil treatment test against upland weeds

In a greenhouse, soybean seeds were sown in 500 cm² ports filled with upland farm soil, and soil containing seeds of barnyard grass (Echinochloa cruss-galli), livid amaranth (Amaranthus lividus L.) and goosefoot (Chenopodium album L.) was put over the soil in the pots in a depth of 1 cm.

One day after the sowing, a test chemical in a predetermined concentration, prepared as in Example 8, was uniformly sprayed over the surface layer of the soil in each of the test pots.

Four weeks after the spraying, the herbicidal effect and the phytotoxicity to crops were examined on the same standards as in Example 8. The results are shown in Table 3.

**Table 3**

| Active compound No. | Amount of active compound (kg/ha) | Herbicidal effect on weeds | | | Phytotoxic effect on soy bean plants |
|---|---|---|---|---|---|
| | | barnyard grass | Livid amaranth | Goosefoot | |
| 9 | 0.125 | 5 | 5 | 5 | 1 |
| | 0.06 | 3 | 4 | 4 | 0 |

| (Known compound) | | | | | |
|---|---|---|---|---|---|
| E-1 | 0.125 | 3 | 4 | 3 | 3 |
| | 0.06 | 1 | 2 | 1 | 1 |
| E-2 | 0.125 | 3 | 5 | 5 | 0 |
| | 0.16 | 1 | 3 | 3 | 0 |

### Example 10

### Herbicidal test by foliage application on upland weeds

In a greenhouse, maize seeds were sown in 500 cm² pots filled with upland farm soil, and soil containing seeds of barnyard grass (Echinochloa cruss-galli), livid amaranth (Amaranthus lividus L.) and goosefoot (Chenopodium album L.) was put over the soil in the ports in a depth of 1 cm.

After sowing, the plants were grown for 14 days and a test chemical in a predetermined concentration, prepared as in Example 8, was uniformly sprayed over the test plants in each of the test ports.

Four weeks after the spraying, the herbicidal effect and the phytotoxicity to crops were examined on the same standards as in Example 8. The results are shown in Table 4.

**Table 4**

| Active compound No. | Amount of active compound (kg/ha) | Herbicidal effect on weeds | | | Phytotoxicity on maize plants |
|---|---|---|---|---|---|
| | | barnyard grass | Livid amaranth | Goosefoot | |
| 4 | 0.125 | 3 | 5 | 5 | 1 |
| | 0.06 | 2 | 4 | 5 | 0 |
| 13 | 0.125 | 4 | 5 | 5 | 1 |
| | 0.06 | 3 | 5 | 5 | 0 |

| (Known compound) | | | | | |
|---|---|---|---|---|---|
| E-1 | 0.125 | 2 | 3 | 3 | 2 |
| | 0.06 | 1 | 2 | 2 | 1 |
| E-2 | 0.125 | 2 | 3 | 3 | 0 |
| | 0.06 | 1 | 2 | 2 | 0 |

## Claims

1) Dimethylmaleinimides of the formula (I) 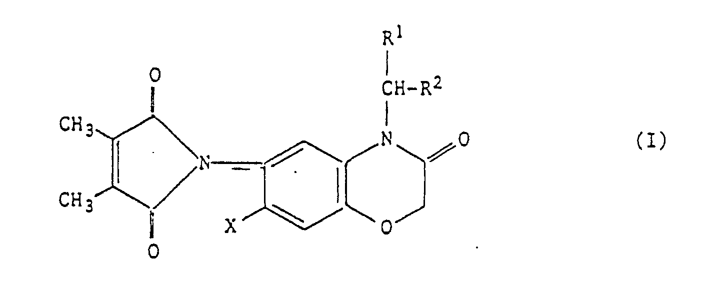 wherein X is hydrogen atom or fluorine atom,
R¹ is hydrogen atom or an alkyl group having 1 to 3 carbon atoms,
R² is a haloalkyl group having 1 to 2 carbon atoms, a halovinyl group, an alkoxyalkoxyalkyl group having 3 to 6 carbon atoms, carbamoyl group, thiocarbamoyl group,
- 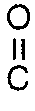 -R³, 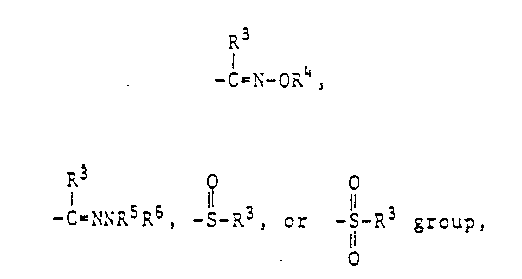 wherein
R³ is an alkyl group having 1 to 4 carbon atoms or a cycloalkyl group having 3 to 6 carbon atoms,
R⁴ is hydrogen atom, an alkyl group having 1 to 4 carbon atoms, an alkenyl group having 2 to 4 carbon atoms, an alkynyl group having 2 to 4 carbon atoms or an alkoxyalkyl group having 2 to 4 carbon atoms, and
R⁵ and R⁶ each are an alkyl group having 1 to 4 carbon atoms.

2) The compounds of the formula (I) according to claim 1 wherein
X is hydrogen atom or fluorine atom,
R¹ is hydrogen atom or methyl group,
R² is 2-chloroethyl, chlorovinyl, methoxyethoxymethyl, carbamoyl, thiocarbamoyl, 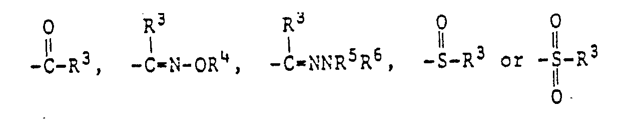 group, wherein R³ is methyl, ethyl or cyclopropyl group,
R⁴ is hydrogen atom, methyl, ethyl, allyl, propargyl or methoxymethyl group, and
R⁵ and R⁶ each are methyl, ethyl, n- or iso-propyl.

3) The compounds of the formula (I) according to cliam 1 wherein
X is fluorine atom,
R¹ is hydrogen atom or methyl group,
R² is 1-chlorovinyl, methoxyethoxymethyl, thiocarbamoyl, 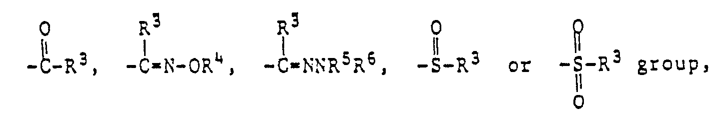 wherein R³ is methyl, ethyl or cyclopropyl group,
R⁴ is hydrogen atom, methyl, ethyl, allyl, propargyl or methoxymethyl group, and
R⁵ and R⁶ each are methyl, ethyl or isopropyl group.

4) The compounds according to claims 1 to 3 wherein
4-(2-chloroallyl)-6-(3,4-dimethylmaleinimido)-7-fluoro-2H-1,4-benzoxazin-3(4H)-one of the formula: 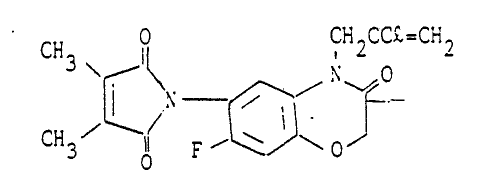 6-(3,4-dimethylmaleinimido)-7-fluoro-4-thiocarbamoylmethyl-2H-1,4-benzoxazin-3(4H)-one of the formula: 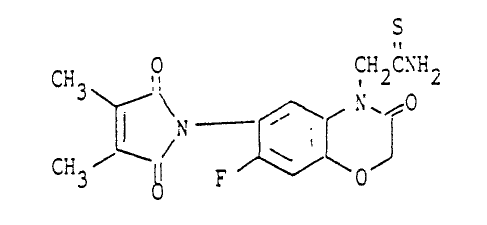 6-(3,4-dimethylmaleinimido)-7-fluoro-4-(2-oxopropyl)-2H-1,4-benzoxazin-3(4H)-one of the formula: 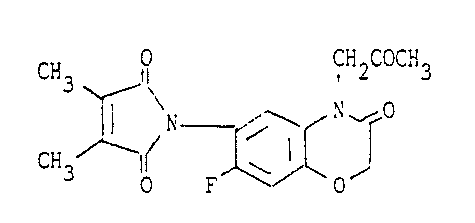 6-(3,4-dimethylmaleinimido)-7-fluoro-4-(2-oxobutyl)-2H-1,4-benzoxazin-3(4H)-one of the formula: 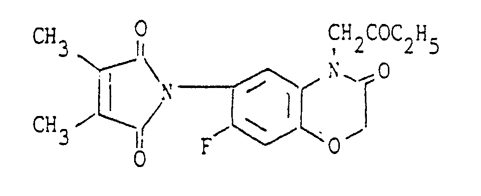 6-(3,4-dimethylmaleinimido)-7-fluoro-4-(2-hydroxyiminopropyl)-2H-1,4-benzoxazin-3(4H)-one of the formula: 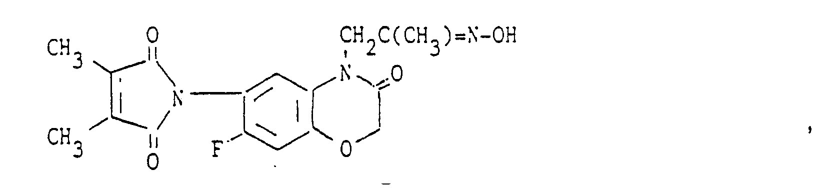 4-[2-(N,N-dimethylhydrazono)propyl]-6-(3,4-dimethylmaleinimido)-7-fluoro-2H-1,4-benzoxazin-3(4H)-one of the formula: 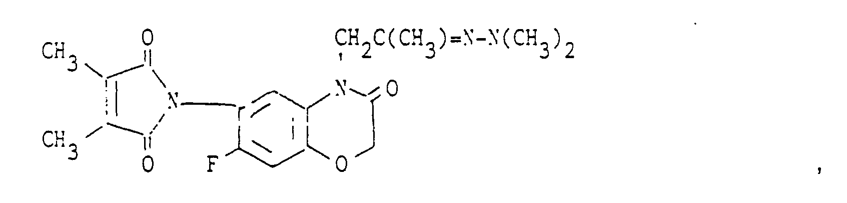 6-(3,4-dimethylmaleinimido)-7-fluoro-4-methylsulfinylmethyl-2H-1,4-benzoxazin-3(4H)-one of the formula: 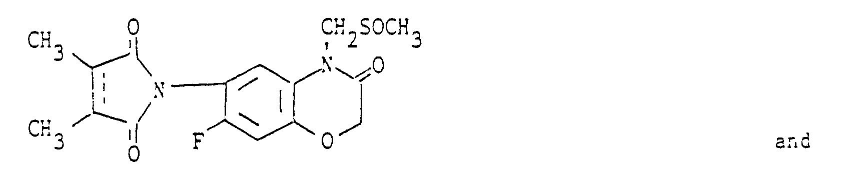 6-(3,4-dimethylmaleinimido)-7-fluoro-4-methylsulfonylmethyl-2H-1,4-benzoxazin-3(4H)-one of the formula:

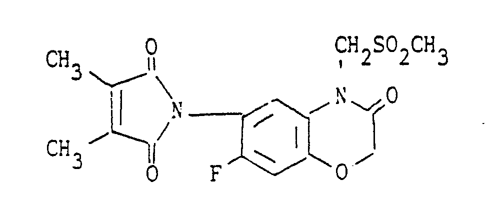

5) Process for the preparation of dimethylmaleinimides of the formula (I) 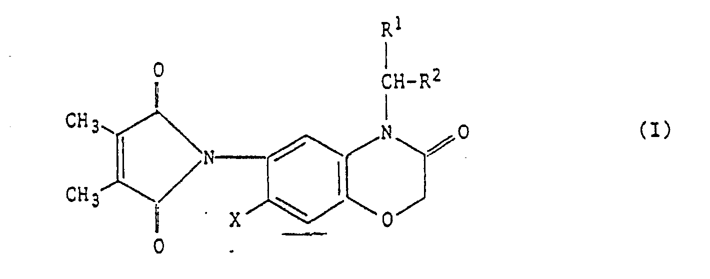 wherein X is hydrogen atom or fluorine atom,
R¹ is hydrogen atom or an alkyl group having 1 to 3 carbon atoms,
R² is a haloalkyl group having 1 to 2 carbon atoms, a halovinyl group, an alkoxyalkoxyalkyl group having 3 to 6 carbon atoms, carbamoyl group, thiocarbamoyl group,
- 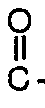 -R³, 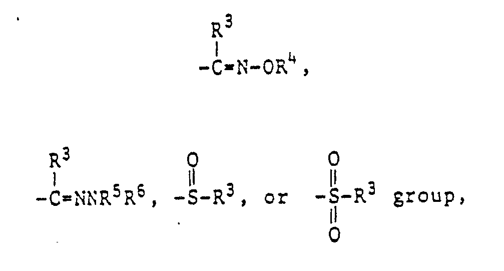 wherein
R³ is an alkyl group having 1 to 4 carbon atoms or a cycloalkyl group having 3 to 6 carbon atoms,
R⁴ is hydrogen atom, an alkyl group having 1 to 4 carbon atoms, an alkenyl group having 2 to 4 carbon atoms, an alkynyl group having 2 to 4 carbon atoms or an alkoxyalkyl group having 2 to 4 carbon atoms, and
R⁵ and R⁶ each are an alkyl group having 1 to 4 carbon atoms, characterised in that
a) compounds of the formula (II) 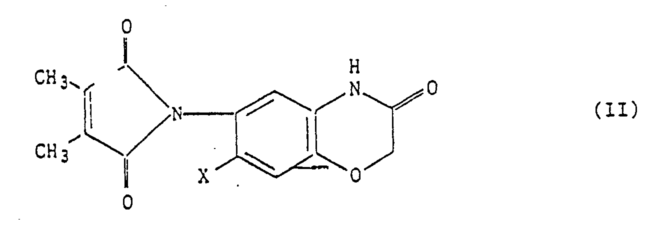 wherein X has the same meaning as above,
are reacted with compounds of the formula (III) 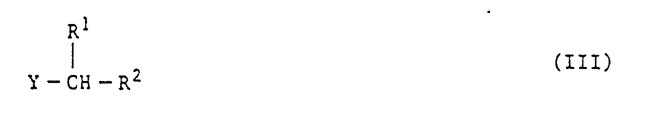 wherein R¹ and R² have the same meanings as above, and Y represents halogen atom or -OSO₂-T, wherein T is methyl, phenyl or tolyl group,
in the presence of inert solvents and if appropriate, in the presence of a base,
or
b) compounds of the formula (IV) 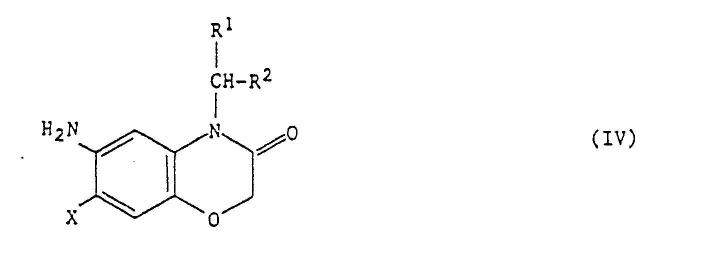 wherein R¹, R² and X have the same meanings as mentioned above, are reacted with 2,3-dimethylmaleic anhydride in the presence of inert solvents,
or
c) in the case where R² is 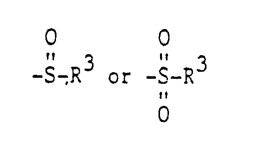 compounds of the formula (V) 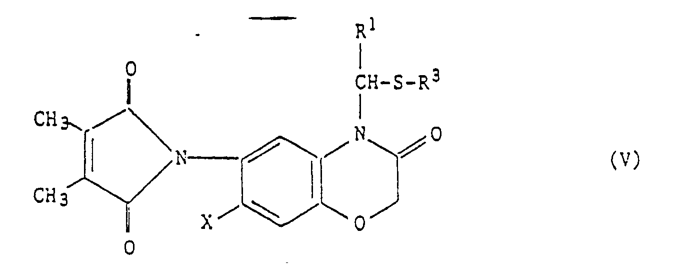 wherein R¹, R³ and X have the same meanings as mentioned above, are oxidized,
or
d) in the case where R² is thiocarbamoyl compounds of the formula (VI) 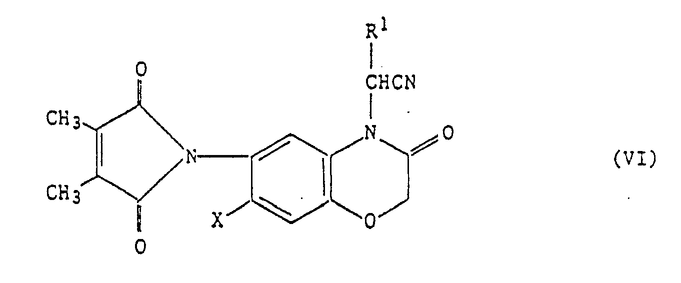 wherein R¹ and X have the same meanings as mentioned above,
are reacted with hydrogen sulfide in the presence of inert solvents,
or
e) in the case where R² is 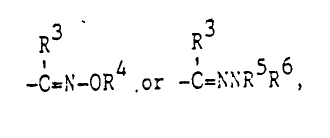 compounds of the formula (VII) 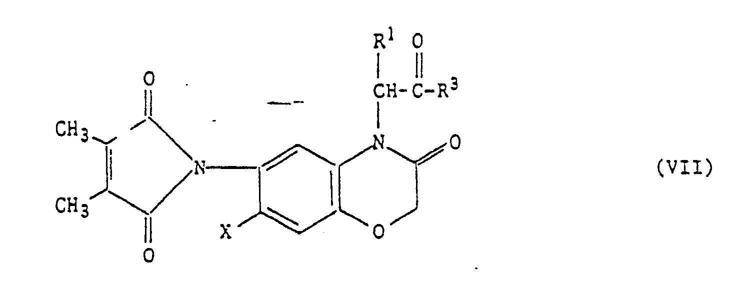 wherein R¹, R³ and X have the same meanings as mentioned above, are reacted with compounds of the formula (VIII)
H₂N-R⁷ (VIII)
wherein R⁷ is -OR⁴ or -NR⁵R⁶ and
R⁴, R⁵ and R⁶ have the same meanings as mentioned above, in the presence of inert solvents,

6) Herbicidal compositions, characterised in that they contain at least one dimethylmaleinimide of the formula (I).according to claims 1 to 5.

7) Process for combating weeds, characterised in that dimethylmaleinimides of the formula (I) according to claims 1 to 5 are allowed to act on weeds and/or their habitat.

8) Use of dimethylmaleinimides of the formula (I) according to claims 1 to 5 for combating weeds.

9) Process for the preparation of herbicidal compositions, characterised in that dimethylmaleinimides of the formula (I) according to claims 1 to 5 are mixed with extenders and/or surface active agents.

10) Compounds of the formula (IX) 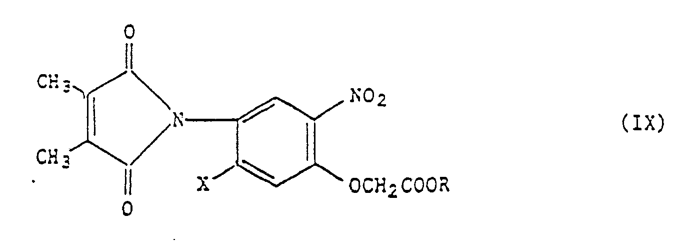 in which
X is hydrogen or fluorine and
R is an alkyl group.

11) Process for the preparation of compounds of the formula (IX) 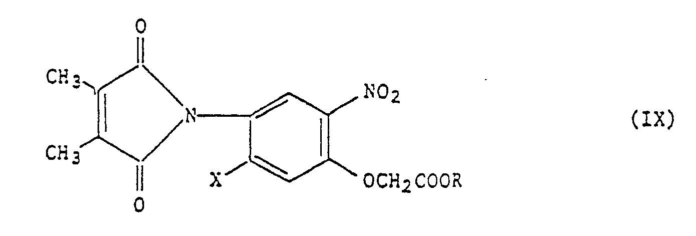 in which
X is hydrogen or fluorine and
R is an alkyl group,
characterized in that compounds of the formula (X) 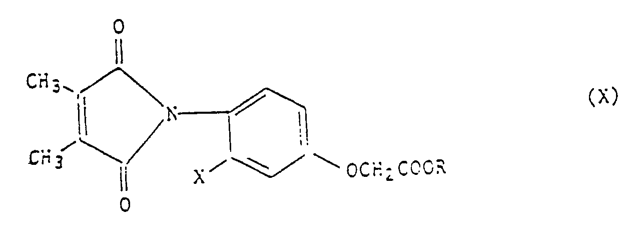 wherein X and R have the same meanings as mentioned above, are nitrated.
